# EUROPEAN PATENT APPLICATION

(11) **EP 4 230 042 A1**
(43) Date of publication of application: **23.08.2023**
(21) Application number: 21877750.6
(22) Date of filing: 08.10.2021
(51) Int. Cl.: A01N 59/08, A01N 59/00, A01P 1/00, A01P 3/00, A61L 9/00, A61L 9/01, A61L 9/015, A61L 9/14

(54) **ANTIPATHOGENIC DRUG, ANTIBACTERIAL AGENT, ANTIVIRAL AGENT, PATHOGEN PROCESSING DEVICE, METHOD FOR PRODUCING ANTIPATHOGENIC DRUG, ANTIBACTERIAL METHOD, VIRUS INACTIVATION METHOD, AND PATHOGEN PROCESSING METHOD**

(30) Priority: 09.10.2020 JP 2020171087
(71) Applicant: Acenet Inc., Tokyo 105-0021 (JP)
(72) Inventor: TAKAMORI Kiyoto, Tokyo 105-0021 (JP); SHIBATA Takekatsu, Tokyo 105-0021 (JP); KONISHI Kiyoshi, Tokyo 105-0021 (JP)
(74) Representative: J A Kemp LLP
(86) International application number: PCT/JP2021/037342
(87) International publication number: WO 2022/075451

(57) **Abstract**

An antipathogenic agent that is highly safe and has high antibacterial and virus inactivation effects. An antipathogenic agent includes: a radical source; and ozone, wherein the radical source includes at least one material selected from the group consisting of halogenous acids, halite ions, and halites, and the radical source and the ozone are brought into contact with at least one target selected from the group consisting of an object containing a pathogen and an environment containing the pathogen, at the same time or at different times. The halogenous acid is a chlorous acid, a bromous acid, an iodous acid, or the like.

## Description

### TECHNICAL FIELD

The present invention relates to an antipathogenic agent, an antibacterial agent, an antiviral agent, a pathogen disposal device, an antipathogenic agent production method, an antibacterial treatment method, a virus inactivation method, and a pathogen disposal method.

### BACKGROUND ART

Diseases caused by infection with bacteria, viruses, and the like have long been problems all over the world. At present, in order to avoid infection, antibacterial treatment or virus inactivation is performed by, for example, spraying an antibacterial agent or a virus inactivation agent on bacteria or viruses. Various antibacterial agents and virus inactivation agents are currently used, and examples thereof include ethanol for disinfection and hypochlorous acid. For example, Patent Literature 1 describes antibacterial treatment of water in swimming pools with hypochlorous acid. Further, for example, Patent Literature 2 describes that an object to be disposed in a chamber is applied with antibacterial treatment using ozone gas.

### Citation List

### Patent Literature

Patent Literature 1: JP 2014-091063 A
Patent Literature 2: JP 2017-164260 A

### SUMMARY OF INVENTION

### Technical Problem

Ethanol exhibits a low antibacterial effect as it volatilizes immediately after being sprayed on hands or the like for antibacterial treatment.

Further, hypochlorous acid is decomposed immediately, so that while it has a temporary antibacterial effect, the hypochlorous acid has a problem in that it cannot exhibit an antibacterial effect stably.

Further, since ozone gas has a strong oxidizing power, it may have an adverse effect on the human body. While there is also a method of reducing the concentration of ozone gas in order to suppress the effect on the human body, this method has a disadvantage of reducing antibacterial effect.

With the foregoing in mind, it is an object of the present invention to provide: an antipathogenic agent that is highly safe and has high antibacterial and virus inactivation effects; an antibacterial agent including the same; an antiviral agent including the same; a pathogen disposal device using the same; a method for producing the same; an antibacterial treatment method using the same; a virus inactivation method using the same; and a pathogen disposal method using the same.

### Solution to Problem

In order to achieve the above object, the present invention provides an antipathogenic agent, including: a radical source; and ozone, wherein the radical source includes at least one selected from the group consisting of halogenous acids, halite ions, and halites, and the radical source and the ozone are brought into contact with at least one of an object or an environment containing a pathogen at the same time or at different times.

The present invention also provides an antibacterial agent, including: the antipathogenic agent according to the present invention.

The present invention also provides an antiviral agent, including: the antipathogenic agent according to the present invention.

The present invention also provides a pathogen disposal device configured to dispose a pathogen with an antipathogenic agent including a radical source and ozone, including: an ozone supply mechanism; a radical source container; and a release mechanism, wherein the ozone supply mechanism is configured to supply ozone, the radical source container is configured to store a radical source, the radical source including at least one selected from the group consisting of halogenous acids, halite ions, and halites, the ozone supply mechanism and the radical source container are configured to release the ozone and the radical source to the outside of the device via the release mechanism, and the ozone and the radical source released by the release mechanism are brought into contact with at least one of an object or an environment containing a pathogen.

The present invention also provides a method for producing an antipathogenic agent including a radical source and ozone, including the steps of: releasing ozone; releasing a radical source; and mixing the ozone and the radical source to prepare the antipathogenic agent, wherein the radical source includes at least one selected from the group consisting of halogenous acids, halite ions, and halites.

The present invention also provides an antibacterial treatment method, including the steps of: producing an agent; and contacting, wherein the agent producing step is a method for producing an antipathogenic agent according to the present invention; and the contacting step brings at least one of the ozone and the radical source in combination or the prepared antipathogenic agent into contact with at least one of an object or an environment containing a pathogen to perform antibacterial treatment.

The present invention also provides a virus inactivation method, including the steps of: producing an agent; and contacting, wherein the agent producing step is a method for producing an antipathogenic agent according to the present invention; and the contacting step brings at least one of the ozone and the radical source in combination or the prepared antipathogenic agent into contact with at least one of an object or an environment containing a pathogen to perform virus inactivation.

The present invention also provides a pathogen disposal method for disposing a pathogen by an antipathogenic agent including a radical source and ozone, including the steps of: releasing the ozone and the radical source simultaneously or separately; preparing the antipathogenic agent by the release; and contacting at least one of the ozone and the radical source in combination or the prepared antipathogenic agent with at least one of an object or an environment containing a pathogen, wherein the radical source includes at least one selected from the group consisting of halogenous acids, halite ions, and halites.

### Advantageous Effects of Invention

The present invention can provide an antipathogenic agent that is highly safe and has high antibacterial and virus inactivation effects, an antibacterial agent including the same, an antiviral agent including the same, a pathogen disposal device using the same, a method for producing the same, an antibacterial treatment method using the same, a virus inactivation method using the same, and a pathogen disposal method using the same.

### BRIEF DESCRIPTION OF DRAWINGS

[FIGs. 1A and 1B] FIGs. 1A and 1B show schematic diagrams illustrating an exemplary configuration of a pathogen disposal device according to the present invention.
[FIG. 2] FIG. 2 is a graph showing the result of measuring the absorbance in the cell after bubbling with a spectrophotometer in Reference Example 2.

### DESCRIPTION OF EMBODIMENTS

The present invention will be described more specifically below with reference to illustrative examples. It is to be noted, however, that the present invention is by no means limited by the following descriptions.

In the present invention, the term "pathogen" is not particularly limited as long as it has DNA or RNA and causes a disease of an organism. The organisms are not particularly limited, and examples thereof include animals (humans, livestock, etc.) and plants. Specific examples of the pathogen include coronaviruses (including, for example, SARS-CoV, the causative virus of SARS, SARS-CoV-2, the causative virus of COVID-19, and MERS-CoV, the causative virus of MERS), influenza virus, norovirus, hepatitis B virus, hepatitis C virus, mycoplasma, *Staphylococcus aureus*, *Streptococcus pneumoniae*, *Haemophilus influenzae*, ESBL-producing bacteria, *Neisseria gonorrhoeae*, *Mycobacterium tuberculosis*, hemolytic streptococci, *Clostridium difficile*, enterobacteria, enterococci, Acinetobacter, *Pseudomonas aeruginosa*, *Staphylococcus aureus*, Campylobacter, Candida, nontyphoidal salmonella, *Salmonella typhi*, Shigella, Group A Streptococcus, Group B Streptococcus, nontuberculous mycobacteria, spirochetes, and fungi.

As used herein, the term "antibacterial" is not limited to suppression of the growth of microorganisms, and should be interpreted in the broadest sense including sterilization, decolonization, disinfection, total sterilization, bacteriostatic, and the like, and is not limited in any sense. The term "sterilization" means, for example, killing of microorganisms. The term "decolonization" means, for example, the removal and reduction of microorganisms. The term "disinfection" means, for example, killing or reducing microorganisms that are pathogenic to an animal such as a human or plant to detoxify them. The term "total sterilization" means, for example, killing of all microorganisms. The term "bacteriostatic" means, for example, inhibiting or preventing the growth of microorganisms.

In the present invention, the term "antivirus" should be interpreted in the broadest sense including decrease of infectivity of a virus, prevention of infection of a virus, inactivation of a virus, inhibition of growth of a virus, and the like, and is not limited in any sense.

In the present invention, when a compound (e.g., ammonium or the like to be described below) has an isomer such as a tautomer or a stereoisomer (e.g., a geometric isomer, a conformer, and an optical isomer), any isomer can be used unless otherwise specified. In addition, in the present invention, when a substance (for example, a halogenous acid, a halite ion, a radical generating catalyst, and the like to be described below) can form a salt, the salt can also be used unless otherwise specified. The salt may be an acid addition salt or a base addition salt. Furthermore, the acid forming the acid addition salt may be an inorganic acid or an organic acid, and the base forming the base addition salt may be an inorganic base or an organic base. Examples of the inorganic acid include, but are not limited to, sulfuric acid, phosphoric acid, hydrofluoric acid, hydrochloric acid, hydrobromic acid, hydriodic acid, hypofluorous acid, hypochlorous acid, hypobromous acid, hypoiodous acid, fluorine acid, chlorous acid, bromous acid, iodous acid, fluoric acid, chloric acid, bromic acid, iodic acid, perfluoric acid, perchloric acid, perbromic acid, and periodic acid. Examples of the organic acid include, but are not limited to, p-toluenesulfonic acid, methanesulfonic acid, oxalic acid, p-bromobenzenesulfonic acid, carbonic acid, succinic acid, citric acid, benzoic acid, and acetic acid. Examples of the inorganic base include, but are not limited to, ammonium hydroxide, alkali metal hydroxides, alkaline earth metal hydroxides, carbonates, and hydrogencarbonates, and more specifically, sodium hydroxide, potassium hydroxide, potassium carbonate, sodium carbonate, sodium hydrogencarbonate, potassium hydrogencarbonate, calcium hydroxide, and calcium carbonate. Examples of the organic base include, but are not limited to, ethanolamine, triethylamine, and tris(hydroxymethyl)aminomethane. The method for producing these salts is not particularly limited, and for example, the compound can be produced by adding the acid or the base as described above to the compound by a suitable known method.

### (1. Antipathogenic agent)

As described above, the antipathogenic agent of the present invention includes a radical source and ozone. In the antipathogenic agent of the present invention, other configuration and conditions are not particularly limited. Hereinafter, the antipathogenic agent of the present invention may be referred to as the "agent of the present invention".

The agent of the present invention is highly safe and has high antibacterial and virus inactivation effects. Thus, the agent of the present invention can be widely used, for example, for antibacterial treatment, deodorizing, and the like. Further, since the agent of the present invention can directly destroy and decompose bacteria and viruses by ozone, for example, resistant bacteria and resistant viruses are not easily generated.

As a result of study, the inventors of the present invention have found that, by reacting ozone with a radical source such as halogenous acids, the ozone becomes oxygen, which is not toxic. In general, since ozone is harmful to the human body (see References 1 and 2 below), ventilation is required after the use of ozone. However, according to the present invention, since ozone can be detoxified as described above, it can be used safely. The inventors of the present invention have also found that the radical source is oxidized by the ozone to become chlorine dioxide and exhibits antibacterial and antiviral effects. Therefore, according to the present invention, when the ozone and the radical source are brought into contact with at least one of an object or an environment containing a pathogen (hereinafter, an object or the like) at the same time, the ozone is immediately detoxified, and the radical source becomes chlorine dioxide, so that the antibacterial and antiviral effects by the chlorine dioxide can be achieved. In other words, in this case, it can also be said that ozone is not used as an antibacterial agent or an antiviral agent but as an oxidizing agent. On the other hand, when the radical source is brought into contact with the object or the like after the ozone is brought into contact with the object or the like, high antibacterial and antiviral effects can be achieved by both the antibacterial and antiviral effects by the ozone and the antibacterial and antiviral effects by chlorine dioxide generated by the radical source.
Reference 1: Hidetomo Himuro, "The Effect of Ozone on Colonic Epithelial Cells" Kurume Medical Journal, 2017, Vol.64, p.75-81 (Reference 1 is herein incorporated by reference).
Reference 2: Nathalie Dyane Miranda Slompo and Gustavo Henrique Ribeiro da Silva, "Disinfection of anaerobic/aerobic sanitary effluent using ozone: Formaldehyde formation" Water environment research, (United States), 2019, Vol.91, No.9 p. 898-905 (Reference 2 is herein incorporated by reference).

The radical source is at least one selected from the group consisting of halogenous acids, halite ions, and halites. The halogenous acid may include at least one selected from the group consisting of chlorous acids, bromous acids, and iodous acids. In particular, the halogenous acid is preferably the chlorous acid. The radical source may be selected as appropriate depending on the use thereof, with consideration given, for example, to the intensity of reactivity of a radical species, etc. One type of the radical sources may be used alone or two or more types of them may be used in combination.

The content of the radical source (e.g., halogenous acids and the like) in the agent of the present invention is not particularly limited, and is, for example, 0.00001% by mass or more, 0.0001% by mass or more, 0.001% by mass or more, 0.01% by mass or more, or 0.1% by mass or more, and 10% by mass or less, 1% by mass or less, or 0.1% by mass or less. The concentration of the radical source preferably is low, because it is considered that the safety level increases as the concentration becomes lower. However, if the concentration of the radical source is too low, the sterilizing effect or the like may not be obtained. From the viewpoint of the sterilizing effect and the like, the concentration of the radical source is not particularly limited, and preferably is set as high as possible.

In the agent of the present invention, when the radical source is in the form of halites, the halite is not particularly limited, and may be an inorganic salt or an organic salt. The inorganic salt and the organic salt are not particularly limited, and specific examples thereof are, for example, as described above.

The radical source (e.g., halogenous acids, and the like) may be, for example, a liquid, a gas, or a solid.

In the agent of the present invention, the ozone may be, for example, a liquid, a gas, or a solid. Specifically, the ozone may be, for example, ozone gas or ozone water. The ozone gas may be any gas containing ozone and may contain other components. Examples of the other components include oxygen, nitrogen, and carbon dioxide. The ozone water may be a solution obtained by dissolving the ozone gas in water, and may contain other components. The other components are, for example, the same as the other components in the ozone gas. In addition, the ozone in the agent of the present invention may include, for example, both the ozone gas and the ozone water. In the agent of the present invention, as the ozone, one type of the ozone gases, the ozone water, and the like may be used alone or two or more types of them may be used in combination.

The content of the ozone in the agent of the present invention is not particularly limited, and is, for example, greater than 0, 100 ppm or less, 50 ppm or less, 25 ppm or less, 15 ppm or less, or 10 ppm or less. The concentration of the ozone preferably is low, because it is considered that the safety level increases as the concentration becomes lower. However, if the concentration of the ozone is too low, the sterilizing effect or the like may not be obtained. From the viewpoint of the sterilizing effect and the like, the concentration of the ozone is not particularly limited, and preferably is set as high as possible.

The agent of the present invention may further include a radical generating catalyst, for example, and the radical generating catalyst may be, for example, a substance that catalyzes generation of radicals from the radical source in the presence of the ozone. However, the agent of the present invention may or may not include the radical generating catalyst. Hereinafter, the radical generating catalyst may be referred to as the "radical generating catalyst of the present invention".

The radical generating catalyst of the present invention may be, for example, an organic substance or an inorganic substance. The organic substance may be, for example, at least one selected from the group consisting of ammonium, amino acids, proteins, peptides, phospholipids, and salts thereof. The inorganic substance may include one or both of metal ions and nonmetal ions. The metal ion may include one or both of typical metal ions and transition metal ions. The inorganic substance may be, for example, at least one selected from the group consisting of alkali earth metal ions, rare earth ions, Mg²⁺, Sc³⁺, Li⁺, Fe²⁺, Fe³⁺, Al³⁺, silicate ions, and borate ions. Examples of the alkali earth metal ion include ions of calcium, strontium, barium, and radium. More specifically, examples of the alkali earth metal ion include Ca²⁺, Sr²⁺, Ba²⁺, and Ra²⁺. Furthermore, the "rare earth metal" is a generic name of a set of seventeen elements, specifically, two elements such as scandium₂₁Sc and yttrium₃₉Y and fifteen elements (lanthanoids) from lanthanum₅₇La to lutetium₇₁Lu. Examples of the rare earth ion include corresponding trivalent cations of the seventeen elements.

The radical generating catalyst may be, for example, at least one selected from the group consisting of CaCl₂, MgCl₂, FeCl₂, FeCl₃, AlCl₃, AlMeCl₂, AlMe₂Cl, BF₃, BPh₃, BMe₃, TiCl₄, SiF₄, and SiCl₄. It is to be noted that the "Ph" indicates a phenyl group and the "Me" indicates a methyl group.

The radical generating catalyst may be, for example, a Lewis acid having a Lewis acidity of 0.4 eV or more. The radical generating catalyst may also be a brained acid, e.g., an acid dissociation constant pKₐ of 5 or more as a brain-sted acid. The upper limit of pKₐ is not particularly limited, but is, for example, 50 or less.

In the radical generating catalyst of the present invention, the radical generating catalyst can be selected appropriately depending on the intended use thereof, with consideration given to the reactivity, acidity, safety, and the like.

In the present invention, although the reason why the ammonium, amino acids, peptides, and phospholipids function as radical generating catalysts is not clear, it is presumably because the ammonium, amino acids, peptides, and phospholipids each have a function as a Lewis acid. In the present invention, the "Lewis acid" refers to, for example, a substance that serves as a Lewis acid with respect to the radical source.

The Lewis acidity of the radical generating catalyst of the present invention is, for example, 0.4 eV or more, 0.5 eV or more, or 0.6 eV or more. The upper limit of the Lewis acidity is not particularly limited, and is, for example, 20 eV or less. In the present invention, a criterion for judging that the Lewis acidity is equal to, greater than, or less than the above-described numerical value is, for example, whether the measured value by any one of the "Lewis acidity measuring method (1)" and the "Lewis acidity measuring method (2)" described below is equal to, greater than, or less than the above-described numerical value.

The Lewis acidity can be measured, for example, by the method described in Ohkubo, K.; Fukuzumi, S. Chem. Eur. J., 2000, 6, 4532, J. Am. Chem. Soc. 2002, 124, 10270-10271 or the method described in J. Org. Chem. 2003, 68, 4720-4726. Specifically, the Lewis acidity can be measured by the following "Lewis acidity measuring method (1)", which is herein incorporated by reference.

### (Lewis acidity measuring method (1))

As to acetonitrile (MeCN) that contains cobalt tetraphenylporphyrin, saturated O₂, and an object whose Lewis acidity is to be measured (e.g., a cation of a metal or the like, represented by Mⁿ⁺ in the following chemical reaction formula (1a)) in the following chemical reaction formula (1a), the change of the ultraviolet-visible absorption spectrum is measured at room temperature. On the basis of the obtained reaction rate constant (k_{cat}), the ΔE value (eV), which is an indicator of the Lewis acidity, can be calculated. The higher the k_{cat}, the stronger the Lewis acidity. Furthermore, the Lewis acidity of an organic compound can be estimated from the energy level of the lowest unoccupied molecular orbital (LUMO) calculated by the quantum chemical calculation. The higher the value at the positive side, the stronger the Lewis acidity.

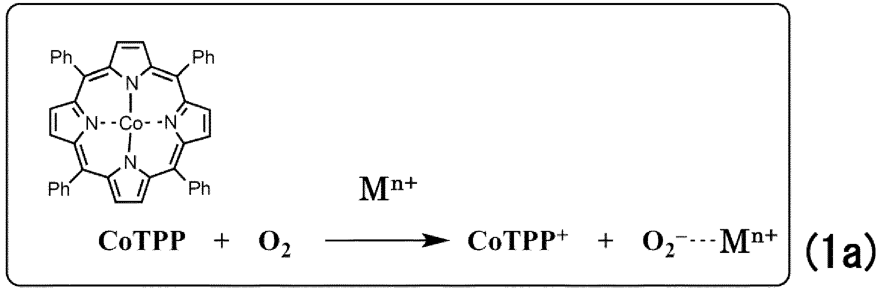

Examples of the reaction rate constant of reaction between CoTPP and oxygen in the presence of a Lewis acid, which is an indicator of the Lewis acidity measured (calculated) by the above-described measurement method, are shown below. In the following table, the numerical value expressed in the unit "k_{cat}, M⁻²s⁻¹" is a reaction rate constant of a reaction between CoTPP and oxygen in the presence of a Lewis acid. The numerical value expressed in the unit "LUMO, eV" is the energy level of LUMO.

| | LUMO, eV | *k*_{cat}, M⁻² s⁻¹ |
|---|---|---|
| benzethonium chloride | -4.12 | 0.24 |
| benzalkonium chloride | -4.02 | 0.18 |
| tetramethylammonium hexafluorophosphate | -3.58 | > 0.1 |
| tetrabutylammonium hexafluorophosphate | -2.07 | > 0.1 |
| ammonium hexafluorophosphate | -5.73 | 20 |

In the present invention, the Lewis acidity may be measured by reducing ubiquinone 1 using ubiquinone 1 (Q1) instead of an oxygen molecule (O₂) to generate an anion radical of ubiquinone 1 in the Lewis acidity measuring method (1). In the following description, such a Lewis acidity measuring method may be referred to as the "Lewis acidity measuring method (2)". In the Lewis acidity measuring method (2), the measurement can be performed in the same manner as in the Lewis acidity measuring method (1), except that ubiquinone 1 (Q1) is used instead of the oxygen molecule (O₂). In the Lewis acidity measuring method (2), similarly to the Lewis acidity measuring method (1), the ΔE value (eV), which is an index of Lewis acidity, can be calculated from the obtained reaction rate constant (k_{cat}). The Lewis acidity measuring method (2) is, for example, described in Ohkubo, K.; Fukuzumi, S. Chem. Eur. J., 2000, 6, 4532, and can be performed according to or based on the method described therein.

The Lewis acidity measuring method (2) can be performed by measuring the reaction rate constant (k_{cat}) with respect to the following chemical reaction formula (1b). 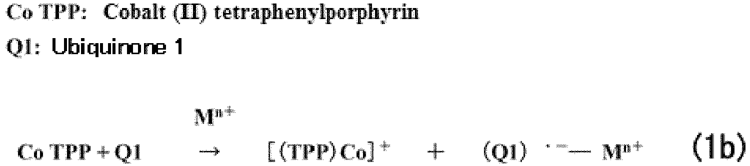 in the chemical formula (1b),
Mⁿ⁺ represents the radical generating catalyst,
CoTPP represents cobalt (II) tetraphenylporphyrin,
Q1 represents ubiquinone 1,
[(TPP)Co]⁺ represents cobalt (III) tetraphenylporphyrin cation, and
(Q1)^{·-} represents an anionic radical of ubiquinone 1.

The Lewis acidity of the radical generating catalyst of the present invention may have a reaction rate constant (k_{cat}) for the chemical reaction formula (1b), i.e., a measured value (K_{obs}) of the reaction rate constant (k_{cat}) measured by the "Lewis acidity measuring method (2)", of, for example, 1.0 × 10⁻⁵S⁻¹ or more, 2.0 × 10⁻⁵S⁻¹ or more, 3.0 × 10⁻⁵S⁻¹ or more, 4.0 × 10⁻⁵S⁻¹ or more, 5.0 × 10⁻⁵S⁻¹ or more, 6.0 × 10⁻⁵S⁻¹ or more, 7.0 × 10⁻⁵S⁻¹ or more, 8.0 × 10⁻⁵S⁻¹ or more, 9.0 × 10⁻⁵S⁻¹ or more, 1.0 × 10⁻⁴S⁻¹ or more, 2.0 × 10⁻⁴S⁻¹ or more, 3.0 × 10⁻⁴S⁻¹ or more, 4.0 × 10⁻⁴S⁻¹ or more, 5.0 × 10⁻⁴S⁻¹ or more, 6.0 × 10⁻⁴S⁻¹ or more, 7.0 × 10⁻⁴S⁻¹ or more, 8.0 × 10⁻⁴S⁻¹ or more, 9.0 × 10⁻⁴S⁻¹ or more, 1.0 × 10⁻³S⁻¹ or more, 2.0 × 10⁻³S⁻¹ or more, 3.0 × 10⁻³S⁻¹ or more, 4.0 × 10⁻³S⁻¹ or more, 5.0 × 10⁻³S⁻¹ or more, 6.0 × 10⁻³S⁻¹ or more, 7.0 × 10⁻³S⁻¹ or more, 8.0 × 10⁻³S⁻¹ or more, 9.0 × 10⁻³S⁻¹ or more, 1.0 × 10⁻²S⁻¹ or more, 2.0 × 10⁻²S⁻¹ or more, 3.0 × 10⁻²S⁻¹ or more, 4.0 × 10⁻²S⁻¹ or more, 5.0 × 10⁻²S⁻¹ or more, 6.0 × 10⁻²S⁻¹ or more, 7.0 × 10⁻²S⁻¹ or more, 8.0 × 10⁻²S⁻¹ or more, or 9.0 × 10⁻²S⁻¹ or more; or 1.0 × 10⁻¹S⁻¹ or less, 9.0 × 10⁻²S⁻¹ or less, 8.0 × 10⁻²S⁻¹ or less, 7.0 × 10⁻²S⁻¹ or less, 6.0 × 10⁻²S⁻¹ or less, 5.0 × 10⁻²S⁻¹ or less, 4.0 × 10⁻²S⁻¹ or less, 3.0 × 10⁻²S⁻¹ or less, 2.0 × 10⁻²S⁻¹ or less, 1.0 × 10⁻²S⁻¹ or less, 9.0 × 10⁻³S⁻¹ or less, 8.0 × 10⁻³S⁻¹ or less, 7.0 × 10⁻³S⁻¹ or less, 6.0 × 10⁻³S⁻¹ or less, 5.0 × 10⁻³S⁻¹ or less, 4.0 × 10⁻³S⁻¹ or less, 3.0 × 10⁻³S⁻¹ or less, 2.0 × 10⁻³S⁻¹ or less, 1.0 × 10⁻³S⁻¹ or less, 9.0 × 10⁻⁴S⁻¹ or less, 8.0 × 10⁻⁴S⁻¹ or less, 7.0 × 10⁻⁴S⁻¹ or less, 6.0 × 10⁻⁴S⁻¹ or less, 5.0 × 10⁻⁴S⁻¹ or less, 4.0 × 10⁻⁴S⁻¹ or less, 3.0 × 10⁻⁴S⁻¹ or less, 2.0 × 10⁻⁴S⁻¹ or less, 1.0 × 10⁻⁴S⁻¹ or less, 9.0 × 10⁻⁵S⁻¹ or less, 8.0 × 10⁻⁵S⁻¹ or less, or 7.0 × 10⁻⁵S⁻¹ or less.

In the radical generating catalyst of the present invention, the ammonium may be, for example, quaternary ammonium, or tertiary, secondary, primary or zero ammonium. The ammonium is not particularly limited, and may be, for example, a nucleic acid base or the like, or an amino acid, a peptide, or the like described below.

The radical generating catalyst of the present invention, may be, for example, a cationic surfactant, which may be a quaternary ammonium-type cationic surfactant. Examples of the quaternary ammonium-type cationic surfactant include benzalkonium chloride, benzethonium chloride, cetylpyridinium chloride, hexadecyltrimethylammonium bromide, dequalinium chloride, edrophonium, didecyldimethylammonium chloride, tetramethylammonium chloride, tetrabutylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, betalain, lecithin, adenine, guanine, cytosine, thymine, uracil, and cholines (e.g., choline chlorides [such as benzoyl choline chloride and a lauroylcholine chloride hydrate], phosphocholine, acetylcholine, choline, dipalmitoylphosphatidylcholine, and choline bitartrate). It is to be noted, however, that, in the radical generating catalyst of the present invention, the quaternary ammonium is not limited to a surfactant.

In the radical generating catalyst of the present invention, the ammonium may be, for example, ammonium represented by the following chemical formula (XI).

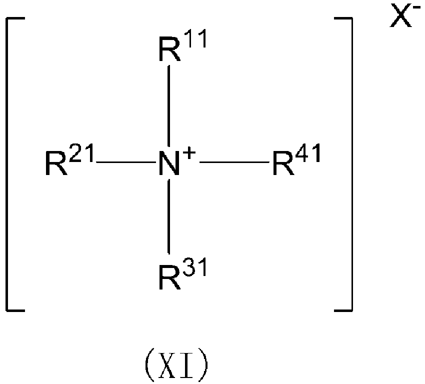

In the chemical formula (XI), R¹¹, R²¹, R³¹, and R⁴¹ are each a hydrogen atom or an aromatic ring, or an alkyl group and the alkyl group may include an ether bond, a carbonyl group, an ester bond, or an amide bond, or an aromatic ring, and R¹¹, R²¹, R³¹, and R⁴¹ may be identical to or different from each other, or two or more of R¹¹, R²¹, R³¹, and R⁴¹ may be integrated to form a ring structure with N⁺ to which they are bonded, and the ring structure may be saturated or unsaturated, aromatic or non-aromatic, and may or may not have one or more substituents, and X⁻ is an anion, and X⁻ is, for example, an anion excluding peroxodisulfate ion. In R ¹¹, R²¹, R³¹, and R⁴¹, the aromatic ring is not particularly limited, and, for example, may or may not contain a heteroatom, and may or may not have a substituent. Examples of the aromatic ring containing a heteroatom (heteroaromatic ring) include a nitrogen-containing aromatic ring, a sulfur-containing aromatic ring, and an oxygen aromatic ring. Examples of the aromatic ring not containing a heteroatom include a benzene ring, a naphthalene ring, an anthracene ring, and a phenanthrene ring. Examples of the heteroaromatic ring include a pyridine ring, a thiophene ring, and a pyrene ring. The nitrogen-containing aromatic ring, for example, may or may not have a positive charge. Examples of the nitrogen-containing aromatic ring having no positive charge include a pyrroline ring, a pyridine ring, a pyridazine ring, a pyrimidine ring, a pyrazine ring, a quinoline ring, an isoquinoline ring, an acridine ring, a 3,4-benzoquinoline ring, a 5,6-benzoquinoline ring, a 6,7-benzoquinoline ring, a 7,8-benzoquinoline ring, a 3,4-benzoisoquinoline ring, a 5,6-benzoisoquinoline ring, a 6,7-benzoisoquinoline ring, and a 7,8-benzoisoquinoline ring. Examples of the nitrogen-containing aromatic ring having a positive charge include a pyrrolinium ring, a pyridinium ring, a pyridazinium ring, a pyrimidinium ring, a pyrazinium ring, a quinolinium ring, an isoquinolinium ring, an acridinium ring, a 3,4-benzoquinolinium ring, a 5,6-benzoquinolinium ring, a 6,7-benzoquinolinium ring, a 7,8-benzoquinolinium ring, a 3,4-benzoisoquinolinium ring, a 5,6-benzoisoquinolinium ring, a 6,7-benzoisoquinolinium ring, and a 7,8-benzoisoquinolinium ring. The oxygen-containing aromatic ring or the sulfur-containing aromatic ring may be, for example, an aromatic ring in which at least one of a carbon atom or a nitrogen atom of the above-described heteroatom-free aromatic ring or nitrogen-containing aromatic ring is substituted with at least one of an oxygen atom and a sulfur atom. In R ¹¹, R²¹, R³¹, and R⁴¹, when the alkyl group or the aromatic ring has a substituent, the substituent is not particularly limited, is optional, and examples thereof include a sulfo group, a nitro group, and a diazo group.

The ammonium salt represented by the chemical formula (XI) may be, for example, ammonium salt represented by the following chemical formula (XII).

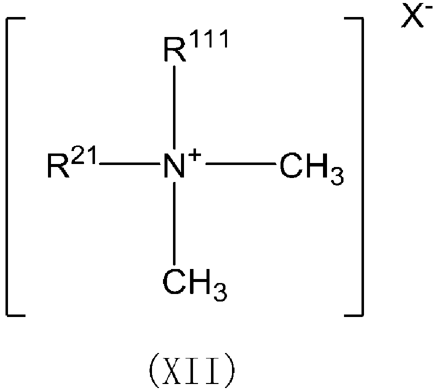

In the chemical formula (XII), R¹¹¹ is an alkyl group having 5 to 40 carbon atoms and may include an ether bond, a ketone (carbonyl group), an ester bond, or an amide bond, substituent, or an aromatic ring, and R²¹ and X⁻ are the same as those in the chemical formula (XI). In R¹¹¹, the aromatic ring is not particularly limited, and, for example, may or may not contain a heteroatom, and may or may not have a substituent. In R¹¹¹, the aromatic ring is not particularly limited, and specific examples are the same as those in R¹¹, R²¹, R³¹, and R⁴¹ of the chemical formula (XI). In R¹¹¹, when the alkyl group or the aromatic ring has a substituent, the substituent is not particularly limited, is optional, and, for example, are the same as those in R¹¹, R²¹, R³¹, and R⁴¹ of the chemical formula (XI).

In the chemical formula (XII), R²¹ may be, for example, a methyl group or a benzyl group. In the benzyl group, one or more hydrogen atoms on the benzene ring may or may not be substituted with any substituent. The substituent may be, for example, an alkyl group, an unsaturated aliphatic hydrocarbon group, an aryl group, a heteroaryl group, a halogen, a hydroxy group (-OH), a mercapto group (-SH), or an alkylthio group (-SR, where R is an alkyl group).

The ammonium salt represented by the chemical formula (XII) may be, for example, ammonium represented by the following chemical formula (XIII).

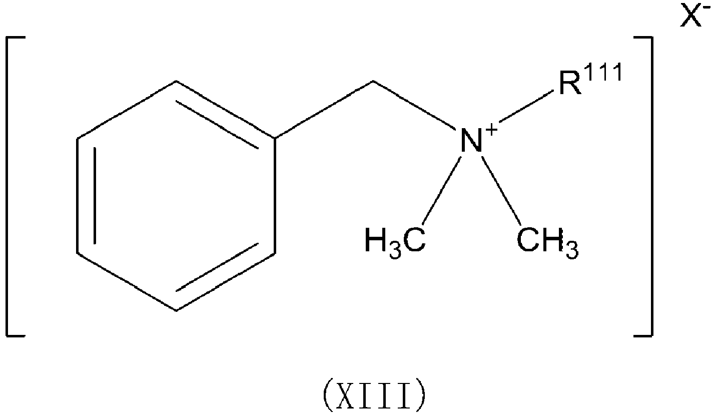

In the chemical formula (XIII), R¹¹¹ and X⁻ are the same as those in the chemical formula (XII).

The ammonium may be, for example, at least one material selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, betalain, lecithin, adenine, guanine, cytosine, thymine, uracil, and cholines. The ammonium may be, for example, benzethonium chloride.

The ammonium salt represented by the chemical formula (XI) may be, for example, ammonium salt represented by the following chemical formula (XIV). 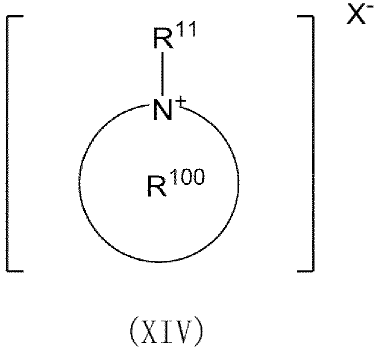 where in the chemical formula (XIV), R¹⁰⁰ may form a ring structure, which may be saturated or unsaturated, aromatic or non-aromatic, and may or may not have one or more substituents, and R¹¹ and X⁻ are the same as those in the chemical formula (XI).

The ammonium salt represented by the chemical formula (XI) may be, for example, ammonium salt represented by the following chemical formula (XV).

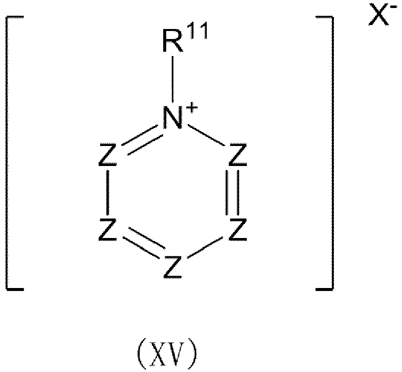

In the chemical formula (XV), Zs are each CH or N, may be identical to or different from each other, and in the case of CH, H may be substituted with a substituent, and R¹¹ and X⁻ are the same as those in the chemical formula (XI).

The ammonium salt represented by the chemical formula (XI) may be, for example, ammonium salt represented by the following chemical formula (XVI).

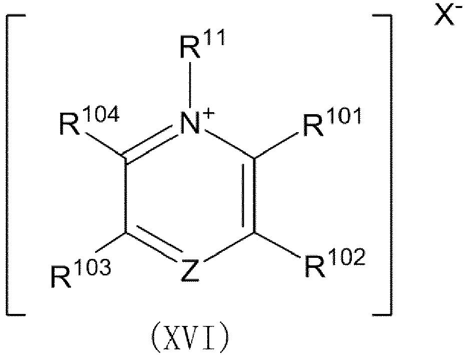

In the chemical formula (XVI), R¹⁰¹, R¹⁰², R¹⁰³, and R¹⁰⁴ are each a hydrogen atom or a substituent, and R¹⁰¹, R¹⁰², R¹⁰³, and R¹⁰⁴ may be identical to or different from each other, or two or more of R¹⁰¹, R¹⁰², R¹⁰³, and R¹⁰⁴ may be integrated to form a ring structure with N⁺ to which they are bonded, and the ring structure may be saturated or unsaturated, aromatic or non-aromatic, and may or may not have one or more substituents, Z is CH or N, and in the case of CH, H may be substituted with a substituent, and R¹¹ and X⁻ are the same as those in the chemical formula (XI).

The ammonium salt represented by the chemical formula (XI) may be, for example, ammonium salt represented by the following chemical formula (XVII).

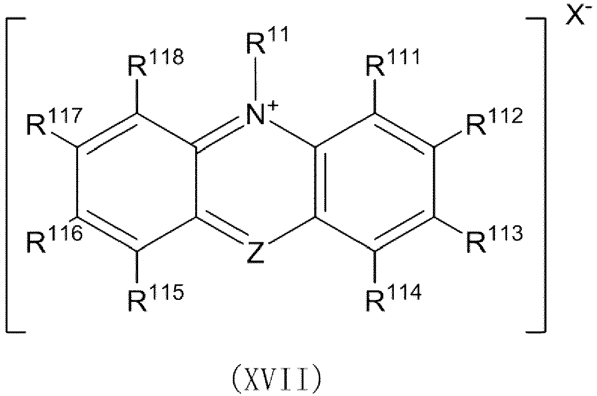

In the chemical formula (XVII), R¹¹¹ to R¹¹⁸ are each a hydrogen atom or a substituent, and may be identical to or different from each other, or two or more of R¹¹¹ to R¹¹⁸ may be integrated to form a ring structure, which may be aromatic or non-aromatic and may or may not have one or more substituents, Z is CH or N, and in the case of CH, H may be substituted with a substituent, and R¹¹ and X⁻ are the same as those in the chemical formula (XI).

The ammonium salt represented by the chemical formula (XI) may be, for example, at least one selected from the group consisting of benzethonium chloride, benzalkonium chloride, hexadecyltrimethylammonium chloride, tetramethylammonium chloride, ammonium chloride, methylammonium chloride, and tetrabutylammonium chloride. It is particularly preferable that the ammonium salt represented by the chemical formula (XII) is benzethonium chloride.

Benzethonium chloride (Bzn⁺Cl⁻) can be, for example, represented by the following chemical formula. Benzalkonium chloride can be, for example, a compound represented by the chemical formula (XIII) where R¹¹¹¹ is an alkyl group having 8 to 18 carbon atoms and X⁻ is a chloride ion.

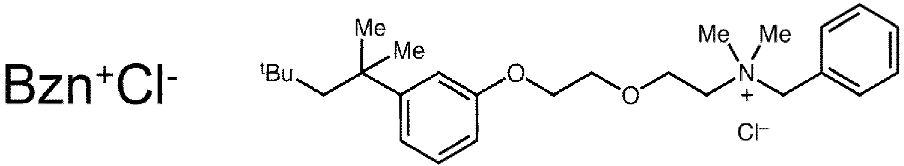

In the chemical formulae (XI), (XII), (XIII), (XIV), (XV), (XVI), and (XVII), X⁻ may be any anion and is not particularly limited. X⁻ is not limited to a monovalent anion, and may be an anion with any valence, such as a divalent anion or a trivalent anion. When the anion is an anion with a plurality of electric charges, such as a divalent anion or a trivalent anion, the number of molecules of the ammonium (monovalent) in each of the chemical formulae (XI), (XII), (XIII), (XIV), (XV), (XVI), and (XVII) is determined by, for example, [the number of molecules of the anion × the valence of the anion] (e.g., when the anion is divalent, the number of molecules of the ammonium (monovalent) is twice the number of molecules of the anion). X⁻ may be, for example, a halogen ion (a fluoride ion, a chloride ion, a bromide ion, or an iodide ion), an acetate ion, a nitrate ion, or a sulfate ion.

In the present invention, the radical generating catalyst is not limited to the chemical formulae (XI), (XII), (XIII), (XIV), (XV), (XVI) and (XVII), and may be ammonium having any structure containing an aromatic ring. The aromatic ring is not particularly limited, and may be, for example, an aromatic ring exemplified in R¹¹, R²¹, R³¹, and R⁴¹ of the chemical formula (XI).

In the present invention, the radical generating catalyst may be, for example, a sulfonic acid amine or ammonium thereof. The sulfonic acid amine is, for example, amine having a sulfonic group (sulfonic acid group) in its molecule. Examples of the sulfonic acid amine include taurine, sulfamic acid, 3-amino-4-hydroxy-1-naphthalenesulfonic acid, sulfamic acid, p-toluidine-2-sulfonic acid, o-anisidine-5-sulfonic acid, direct blue 14, 3- [N, N-bis (2-hydroxyethyl)amino]-2-hydroxypropanesulfonic acid, 3-[(3-colamidopropyl)dimethylammonio]-1-propanesulfonate, aminomethanesulfonic acid, 3-sulfopropylamine, 2-aminobenzenesulfonic acid, R(+)-3-aminotetrahydrofuran, toluene, 4-amino-5-hydroxy-1,7-naphthalenedisulfonic acid, N-(2-acetamido)-2-aminoethanesulfonic acid, 4'-amino-3'-methoxyazobenzene-3-sodium sulfonate, Lapatinib ditosylate, N-tris (hydroxymethyl) methyl-2-aminoethanesulfonic acid, 8-amino-1,3,6-naphthalenetrisulfonic acid disodium hydrate, 1-aminonaphthalene-2-sulfonic acid, (2S,3S)-3-Amino-2-methyl-4-oxo-1-azetidinesulfonic acid, sodium 3- (1-naphthylamino) propanesulfonate, 3-methyl-4-aminobenzenesulfonic acid, sodium 3- Cyclohexylamino-2-hydroxypropanesulfonic acid, sodium N-tris (hydroxymethyl) methyl-2-aminoethanesulfonic acid, 4-amino-1-naphthalenesulfonic acid, sodium sulfamate, tricaine, sodium sulfanilate, 1,4-phenylenediamine 2-sulfonic acid, p- anisidine-2-sulfonic acid, 6-amino-1-naphthalenesulfonic acid, 3,4-diaminobenzene sulfonic acid, 3-amino-4-chlorobenzene sulfonic acid, 3-[(4-Amino-3-methylphenyl) azo] benzenesulfonic acid, 3-amino-4-hydroxy-5-nitrobenzenesulfonic acid, 5-amino-6-hydroxy-3-nitrobenzenesulfonic acid, 4-acetamide-2-Aminobenzenesulfonic acid hydrate, 2-aminophenol-4-sulfonic acid, 1-amino-2-methoxy-5-methyl-4-benzenesulfonic acid, dansylic acid, Sulfamic acid [(1S, 2S, 4R)-4- [4-[[(1S)-2,3-dihydro-1H-inden-1-yl] amino]-7H-pyrrolo [2,3-d] pyrimidin-7-yl]-2- hydroxycyclopropyl] methyl ester, 5-sulfo-4'-diethylamino-2,2' dihydroxyazobenzene, 2-aminonaphthalene-6,8-disulfonic acid, sodium 2- [N,N-bis (2-hydroxyethyl) amino]-1-ethanesulfonate, 3-acetyl-2- (methylaminosulfonyl) thiophene, sodium 4-amino-2-chlorotoluene-5-sulfonate, 5- (3-amino-5-oxo-2-pyrazolin-1-yl)-2-phenoxybenzenesulfonic acid, potassium sulfamate, P-aminobenzene monosulfonic acid, 3-[(3-Cholamidopropyl) dimethylamino]-2-hydroxy-1-propanesulfonate, 3-amino-2,7-naphthalenes disulfonic acid monosodium, 3- [N, N-bis (hydroxyethyl) amino]-2-hydroxypropanesulfonic acid sodium salt, di (amidosulfuric acid) cobalt (II), 3- (4-amino-3-methoxyphenylazo) benzenesulfonic acid, Nickel (II) sulfamate tetrahydrate, sodium 2,4-diaminobenzenesulfonate, 5-amino-2-chlorotoluene-4-sulfonic acid, 2,5-dichlorosulfanilic acid, 4-methylbenzenesulfonic acid, APTS (aminopyrenetrisulfonic acid), 4'-aminoazobenzene-3-sulfonic acid, Pontacyl carmine 2B, p-anisidine-3-sulfonic acid, 4,4'-bis (4-amino-1-naphthylazo)- 2,2'-stilbenesulfonic acid, 3-aminonaphthalene-8-hydroxy-4, 6-disulfonic acid, sodium 4-amino-1,5-naphthalenedisulfonate, sodium 4-aminoazobenzene-4'-sulfonate, 5-amino-2-methylbenzenesulfonic acid, disodium 7-amino-1,3-naphthalene disulfonate, alizarin safilol SE, sodium 7-amino-2-naphthalenesulfonate, 6-amino-5-bromopyridine-3-sulfonic acid, 2-aminoethanethiol p-toluenesulfonate, sodium 2-amino 1-naphthalenesulfonate, 6-amino-1,3-naphthalenedisulfonic acid disodium hydrate, N,N,N',N'-tetraethylsulfamide, 5-amino-2-ethoxybenzenesulfonic acid, 3,5-diamino-2,4,6-trimethylbenzenes Phosphonic acid, 7-amino-1-naphthalenesulfonic acid, sulfamic acid, guanidine, 2-amino-5-nitrobenzenesulfonic acid, nickel (II) diamide sulfate, 4-amino-4'-nitrostilbene-2,2'-disulfonic acid disodium, aniline-2,5-disulfonic acid monosodium, 5-amino-1-naphthol-3-sulfonic acid hydrate, 2,5-dichlorosulfanilic acid sodium salt, 6-aminohexanoic acid hexyl p-toluenesulfonate, rac- (R*)-2- (4-chlorophenyl)-3-amino-1-propanesulfonic acid, 2- (N,N-dipropyl) amino anisole-4-Sulfonic acid, 2-amino-4-chlorophenol-6-sulfonic acid, 6-amino-1,3-naphthalenedisulfonic acid, 5,10,15,20-tetrakis [4-(trimethylammonio) phenyl]- 21H,-23H-porphine tetratosylate, 5-amino-2-[(4-aminophenyl) amino] benzenesulfonic acid, 4-amino-3-chlorobenzenesulfonic acid, 2-aminobenzenesulfonic acid phenyl ester, 4- Acetylamino-4'-isothiocyanatostilbene-2,2'-disulfonic acid disodium salt, (S)-3-Aamino-2-oxetanone P-toluenesulfonic acid salt, 5-acetylamino-4-hydroxy-2,7-naphthalenedisulfonic acid disodium salt, 2-phenylamino-5-aminobenzenesulfonic acid, sodium 4-octadecylamino-4- oxo-2-[(sodiooxy)sulfonyl]butanoate, and 3,5-diamino-4-methylbenzenesulfonic acid.

The radical generating catalyst of the present invention may be, for example, nicotinic amine or ammonium thereof. The nicotinic amine is, for example, amine having a ring structure in a molecule, and the ring structure has a nicotine skeleton. Examples of the nicotinic amine include nicotinamide and alkaloid.

The radical generating catalyst of the present invention may be, for example, nitrite amine or nitrite ammonium. The nitrite amine or nitrite ammonium is, for example, a compound obtained by reacting amine with nitrous acid or a nitrous acid derivative. Examples of the nitrite amine or nitrite ammonium include diazo compounds, diazonium salts, N-nitroso compounds, and C-nitroso compounds.

In the radical generating catalyst of the present invention, the ammonium may include a plurality of ammonium structures (N⁺) in one molecule. Further, the ammonium may form, for example, a dimer, trimer, or the like by association of a plurality of molecules through a π electron interaction.

In the radical generating catalyst of the present invention, the amino acid is not particularly limited. The amino acid may contain, for example, at least one of both an amino group or an imino group and a carboxy group in the molecule. The amino acid may be, for example, an α-amino acid, a β-amino acid, a γ-amino acid, or any other amino acid. The amino acid may be, for example, an amino acid constituting protein, and specifically may be at least one selected from the group consisting of, for example, glycine, alanine, valine, leucine, isoleucine, serine, threonine, aspartic acid, glutamic acid, asparagine, glutamine, lysine, hydroxylysine, arginine, cysteine, cystine, methionine, phenylalanine, tyrosine, tryptophan, histidine, proline, and 4-hydroxyproline.

In the radical generating catalyst of the present invention, the peptide is not particularly limited. The peptide may be, for example, one in which two or more of the amino acid molecules are linked by a peptide bond. The peptide may be, for example, at least one of oxidized glutathione (GSSG) and reduced glutathione (GSH).

In the radical generating catalyst of the present invention, the phospholipid is not particularly limited. The phospholipid may be, for example, a lipid containing phosphorus atoms in the molecule, and may be, for example, a lipid containing a phosphate ester bond (P-O-C) in the molecule. The phospholipid may or may not have, for example, at least one of an amino group, an imino group, an ammonium group, and an iminium group in a molecule. The phospholipid may be, for example, at least one selected from the group consisting of phosphatidylserine, phosphatidylcholine, phosphatidic acid, phosphatidylethanolamine, phosphatidylglycerol, and cardiolipin.

The content of the radical generating catalyst of the present invention in the agent of the present invention is not particularly limited, and may be, for example, 0.1% by mass or more, 1% by mass or more, 10% by mass or more, 25% by mass or more, or 50% by mass or more, and may be, for example, 90% by mass or less, 75% by mass or less, or 60% by mass or less with respect to the total mass of the agent of the present invention. One type of radical generating catalysts of the present invention may be used alone or two or more types of them may be used in combination.

In the agent of the present invention, at least one of the radical source or the ozone may be, for example, dissolved in a solvent. The radical generating catalyst may be, for example, dissolved in the solvent. The solvent may include, for example, at least one of water or an organic solvent. In the agent of the present invention, for example, it is preferable to use water as the solvent from the viewpoint of safety, cost, and the like. The water is not particularly limited, and is preferably, for example, purified water, ion-exchanged water, or pure water. The organic solvent may be, for example, ketone such as acetone, a nitrile solvent such as acetonitrile, or an alcohol solvent such as ethanol, acetic acid solvents, sulfuric acid solvents, and the like. One type of the solvents may be used alone or two or more types of them may be used in combination. The acetic acid solvent and the sulfuric acid solvent may be obtained, for example, by dissolving acetic acid and sulfuric acid in water, respectively. These solvents each serve as, for example, a solvent as well as a Lewis acid or a Brønsted acid. The type of the solvent may be selected as appropriate, for example, depending on the solubility of the solutes (e.g., the radical generating catalyst, the radical source, and the like) etc.. The solvent may be, for example, a mixture of water and an organic solvent. However, the agent of the present invention may or may not include at least one of water or an organic solvent.

The agent of the present invention may include components other than the radical generating catalyst, water, and organic solvent. Examples of the other component include, but are not limited to, a pH adjuster, a buffer, and the like, and one type of the components may be used alone or two or more types of them may be used in combination. The content of the other components in the oral care agent of the present invention is not particularly limited, and may be, for example, 0.1% by mass or more, 1% by mass or more, 10% by mass or more, 25% by mass or more, or 50% by mass or more, and may be, for example, 90% by mass or less, 75% by mass or less, or 60% by mass or less with respect to the total mass of the oral care agent of the present invention.

In the agent of the present invention, the radical source and the ozone may be substances to be brought into contact with at least one of an object or an environment containing a pathogen (hereinafter, also referred to as an object or the like) at the same time or at different times. The contact method is not particularly limited, and the radical source and the ozone may be brought into contact with the object or the like by, for example, spraying, coating, or the like. The object is not particularly limited, and examples thereof include daily necessities, medical instruments, fabric products such as clothing, ornaments such as accessories, furniture such as walls, floors, tables, and the like, in-car (medical vehicle) operating rooms, hospital rooms, wards, contaminant disposal facilities, (garbage, dirt, and blood) disposal vehicles, meat factories, waterworks facilities, sewerage disposal facilities, and morgues. For example, bereaved family are not allowed to visit the bodies of the deceased person due to viral or bacterial infectious diseases, in order to prevent infection. However, if viruses, bacteria, and the like are inactivated by the present invention, the bereaved family can visit the bodies. Examples of the environment include space, water, and soil. Further, the radical source and the ozone may be brought into contact with the object or the like by immersing the object in a solution containing the radical source and the ozone. For example, it is preferable that the ozone first be brought into contact with the object or the like and then the radical source be brought into contact with the object or the like. As a result, as described above, the ozone can be efficiently detoxified. At least one of the radical source or the ozone may be, for example, brought into contact with the object or the like a plurality of times. Specifically, for example, both the radical source and the ozone may be brought into contact with the object or the like a plurality of times, the radical source and the ozone may be alternately brought into contact with the object or the like a plurality of times, or either one of the radical source or the ozone may be brought into contact with the object or the like a plurality of times. More specifically, it is preferable that at least one of the radical source or the ozone be sprayed into a mist to be brought into contact with the object or the like, for example, at the same time or at different times. When the ozone is ozone gas, for example, the radical source may be sprayed in the presence of the ozone gas. When the ozone is ozone water, for example, the ozone water and the radical source may be sprayed at the same time or at different times. When the ozone water and the radical source are sprayed at different times, it is preferable to spray the radical source after spraying the ozone water, for example, as described above.

The pathogen is not particularly limited, and may be, for example, an infectious pathogen. The infection refers to a nature of a microorganism or a virus to stably grow in a host. The microorganism means, for example, a prokaryotic bacterium, a eukaryotic fungus, or the like. Examples of the infectious pathogens are, for example, the same as those of the pathogens described above.

The pH of the agent of the present invention is not particularly limited, and may be, for example, 2 or more, 3 or more, 4 or more, 5 or more, 7 or more, 9 or more, or 10 or more. The pH of the agent of the present invention may be, for example, 13 or less, 12 or less, 11 or less, 10 or less, 9 or less, 8 or less, 7 or less, 6 or less, or 5 or less.

The agent of the present invention may be, for example, liquid, solid, or semi-solid. The agent of the present invention may also be acidic, non-acidic, basic, non-basic, neutral or non-neutral.

As described above, the agent of the present invention is highly safe and has high antibacterial and antiviral effects. The agent of the present invention can exert, for example, a deodorizing action or the like to be described below. Therefore, the agent of the present invention is useful, for example, for improving quality of life (QOL). The agent of the present invention may be, for example, used for prophylactic purposes.

As described above, ethanol volatilizes immediately and is therefore not suitable for use by spraying. Since hypochlorous acid generates toxic chlorine gas, if it is used by spraying, there is a risk that a human or other organism will inhale the chlorine gas. However, the agent of the present invention can be safely used even when it is used by spraying. In addition, as compared to the case where the specific environment (e.g., operating room) is subjected to antibacterial treatment and virus inactivation using only ozone, the present invention can significantly reduce the period of time that allows access to the specific environment.

### (2. Antipathogenic agent production method, etc.)

A method for producing an antipathogenic agent according to the present invention, as described above, is a method of using an antipathogenic agent including a radical source and ozone, including the steps of: releasing ozone; releasing a radical source; and mixing. In the method of using the antipathogenic agent of the present invention, other configurations and conditions are not particularly limited. Further, other configurations and conditions in the antipathogenic agent are not particularly limited, and specifically, for example, according to the antipathogenic agent described above. Hereinafter, a method for producing an antipathogenic agent of the present invention may be referred to as the "production method of the present invention".

In the production method of the present invention, first, ozone is released by the ozone releasing step. The ozone is, for example, the same as described above, and may be ozone gas or ozone water. Further, the production method of the present invention may include, for example, a step of generating ozone prior to the ozone releasing step. For generation of the ozone gas, for example, a known method such as an electrical discharge, an electrolysis method, or an ultraviolet lamp method can be used. For the generation of ozone water, for example, a known method such as a bubble dissolution method, a diaphragm dissolution method, or a packed bed dissolving method can be used. The ozone generating step may generate, for example, both the ozone gas and the ozone water.

The content of the ozone generated by the ozone generating step is not particularly limited.

The amount of the ozone released by the ozone releasing step is not particularly limited. There is also no particular limitation on the interval of release. The duration of the release is also not particularly limited.

Next, the radical source releasing step releases a radical source. The radical source is, for example, the same as described above. Specifically, the radical source includes at least one selected from the group consisting of halogenous acids, halite ions, and halites.

The amount of the radical source released by the radical source releasing step is not particularly limited. There is also no particular limitation on the interval of release. The duration of the release is also not particularly limited.

It is preferable from the viewpoint of reactivity and the like that at least one of the radical source or the ozone be dissolved, for example, in a solvent. The solvent is the same as described above.

At least one of the ozone releasing step or the radical source releasing step may spray at least one of the ozone or the radical source into a mist to release. Specifically, for example, the same as described above.

Then, the ozone and the radical source are mixed by the mixing step to prepare the antipathogenic agent.

The production method of the present invention may further include, for example, a step of releasing a radical generating catalyst. The radical generating catalyst releasing step is a step of releasing a radical generating catalyst. The radical generating catalyst is, for example, the same as described above. When the production method of the present invention includes the radical generating catalyst releasing step, the mixing step further mixes the radical source and the radical generating catalyst. Specifically, for example, the mixing of the radical source and the radical generating catalyst is preferably performed prior to the mixing of the ozone and the radical source.

The amount of the radical generating catalyst released by the radical generating catalyst releasing step is not particularly limited. There is also no particular limitation on the interval of release. The duration of the release is also not particularly limited.

The concentration of the radical generating catalyst is not particularly limited, and may be appropriately set depending on, for example, types of the reactants (raw materials) and the target products.

The mixture (antipathogenic agent) obtained by the mixing step may or may not further contain any material other than the ozone and the radical source. The mixture is, for example, a gas. Specifically, the mixture is, for example, chlorine dioxide or the like. In particular, it has been reported that chlorine dioxide has no adverse effect on organisms but has antibacterial and antiviral effects (see References 3 and 4 below). It is also known that chlorine dioxide has a deodorizing effect or the like.
Reference 3: Norio Ogata. et al., "Generation and Measurement of Chlorine Dioxide Gas at Extremely Low Concentrations in a Living Room: Implications for Preventing Airborne Microbial Infectious Diseases" Pharmacology, (Switzerland"), 2017, Vol.99, p. 114-120 (Reference 3 is herein incorporated by reference).
Reference 4: Norio Ogata. et al., "Inactivation of Airborne Bacteria and Viruses Using Extremely Low Concentrations of Chlorine Dioxide Gas" Pharmacology, (Switzerland"), 2016, Vol.97, p.301-306 (Reference 4 is herein incorporated by reference).

The method of using the agent of the present invention is not particularly limited, and for example, it is preferable to use the agent in a certain space by spraying the agent into a mist using a humidifier, an atomizer, a sprayer, or the like in order to efficiently sterilize the agent. The agent of the present invention is not limited to, for example, human use, and can also be used for animals other than human. Specifically, it can be used, for example, for deodorizing animals and for preventing infectious diseases such as avian influenza and swine influenza. Examples of the use of the agent for animals other than human include those described for human (including for human body). Further, the agent of the present invention may be used as an agent for use in agriculture and livestock industry. The agent for use in agriculture and livestock industry can be used, for example, as an agent for use in agriculture, an agent for use in livestock industry, or the like. The agent for use in agriculture can be used as, for example, a bactericide for use in agriculture, an antiviral agent for use in agriculture, a deodorizer for use in agriculture, an insectcide for use in agriculture, a repellent for use in agriculture, or a soil conditioner for use in agriculture. The agent for use in livestock industry can be used as, for example, a bactericide for use in livestock industry, an antiviral agent for use in livestock industry, a deodorizer for use in livestock industry, an insecticide for use in livestock industry, a repellent for use in livestock industry, or a soil conditioners for use in livestock industry. The agent for use in agriculture and livestock industry may be, for example, applied to one use or two or more uses.

### (3. Antibacterial agent and antibacterial treatment method)

The agent of the present invention can be used, for example, as an antibacterial agent.

The antibacterial treatment method of the present invention includes the production method of the present invention and the contacting step as described above. The contacting step is a step of bringing at least one of the ozone and the radical source in combination or the prepared antipathogenic agent into contact with at least one of an object or an environment containing a pathogen. The antibacterial treatment method of the present invention can perform antibacterial treatment by the contact.

Although various types of substances have been used as an antibacterial agent conventionally, the antibacterial effects of these substances are not sufficient. Some of them can exhibit enhanced antibacterial effects by increasing their concentrations. However, this poses a problem in safety. An antibacterial agent using the agent of the present invention and an antibacterial treatment method of the present invention can exhibit a sufficient antibacterial effect while the concentration of the agent is low and is highly safe.

### (4. Antiviral agent and virus inactivation method)

The agent of the present invention can be used, for example, as an antiviral agent.

The virus inactivation method of the present invention includes the production method of the present invention and the contacting step as described above. The contacting step is a step of bringing at least one of the ozone and the radical source in combination or the prepared antipathogenic agent into contact with at least one of an object or an environment containing a pathogen. The virus inactivation method of the present invention can perform virus inactivation by the contact.

Although various types of substances have been used as an antiviral agent conventionally, the antiviral effects of these substances are not sufficient. Some of them can exhibit enhanced antiviral effects by increasing their concentrations. However, it poses a problem in safety. An antiviral agent using the agent of the present invention and an antibacterial treatment method of the present invention can exhibit a sufficient antiviral effect while the concentration of the agent is low and is highly safe.

### (5. Deodorizer and deodorization method)

The agent of the present invention can be used, for example, as a deodorizer. The deodorization method of the present invention includes the production method of the present invention and the contacting step. The contacting step is a step of bringing at least one of the ozone and the radical source in combination or the prepared antipathogenic agent into contact with at least one of an object or an environment containing a pathogen. The deodorization method of the present invention can perform deodorization by the contact. The object and the environment may or may not contain a pathogen. Commonly used antibacterial agents, such as ethanol, do not have a deodorizing effect. While ozone has a deodorizing effect, the safety level thereof is very low. Some other commercially available products purport to have antibacterial and deodorizing effects. For example, there are commercially available products purporting to exhibit antibacterial and deodorizing effects by spraying them onto clothes directly or spraying them in rooms, toilets, cars, or the like. Such commercially available products typically contain a quaternary ammonium salt as an antibacterial component. However, a commonly used quaternary ammonium salt is not used in combination with a radical source (e.g., a halogenous acid or the like). Thus, in many cases, a sufficient antibacterial effect cannot be obtained unless the quaternary ammonium salt is contained at a high concentration, and this causes a problem of surface tackiness or the like after use. Further, since the quaternary ammonium salt does not have a deodorizing effect, the commercially available products contain a deodorant component as an additional component. While cyclodextrin typically is used as the deodorant component, the cyclodextrin is incapable of decomposing components causing offensive odors. The cyclodextrin merely masks the components causing offensive odors and cannot eliminate the offensive odors themselves. In contrast, the deodorizer containing the agent of the present invention and the deodorization method of the present invention, which have the above-described action mechanism, have a high antibacterial effect, for example, and also, is capable of decomposing substances that cause offensive odors and thus has, for example, a high deodorizing effect.

### (5. Pathogen disposal device and pathogen disposal method)

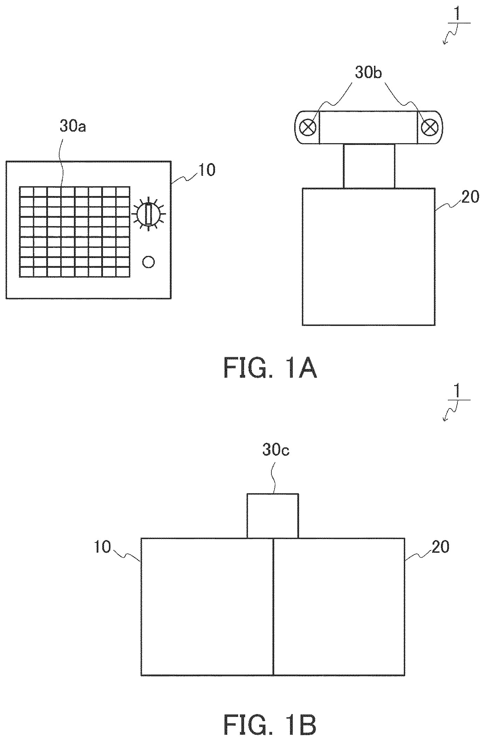

FIGs. 1A and 1B show an exemplary configuration of a pathogen disposal device 1 using the antipathogenic agent of the present invention. Hereinafter, the pathogen disposal device using the antipathogenic agent of the present invention may be referred to as the "pathogen disposal device of the present invention". As shown in FIGs. 1A and 1B, the pathogen disposal device 1 of the present invention is a pathogen disposal device configured to dispose a pathogen by an antipathogenic agent including a radical source and ozone, including an ozone supply mechanism 10, a radical source container 20, and a release mechanism 30 (30a, 30b, or 30c in FIGs. 1A and 1B). The antipathogenic agent is not particularly limited, and is, for example, the antipathogenic agent of the present invention described above. FIG. 1A shows an example of the pathogen disposal device 1 including release mechanisms 30a and a 30b corresponding to the ozone supply mechanism 10 and the radical source container 20, respectively, and FIG. 1B shows an example of the pathogen disposal device 1 in which the ozone supply mechanism 10 and the radical source container 20 share one release mechanism 30c. The ozone supply mechanism 10 and the radical source container 20 may be independent devices as shown in FIG. 1A, or may be an integrally molded device as shown in FIG. 1B. The ozone supply mechanism 10, the radical source container 20, and the release mechanism 30 will be described in detail below but not limited to the following forms.

The ozone supply mechanism 10 supplies ozone. Specifically, the ozone supply mechanism 10 may include, for example, at least one of ozone generation mechanisms.

The ozone generation mechanism generates ozone. Specifically, the ozone generation mechanism is, for example, a device configured to generate ozone gas, ozone water, and the like by the above-described method. The pathogen disposal device 1 may include, for example, both the ozone generation mechanism configured to generate ozone gas and the ozone generation mechanism configured to generate ozone.

The radical source container 20 is a container configured to store a radical source. The radical source is, for example, the same as described above. The material of the radical source container 20 is not particularly limited as long as it can store the radical source. Specific examples of the radical source container 20 include light-shielding chemical tanks made of resin and containers made of resin such as polyethylene terephthalate (PET). The size of the radical source container 20 is not particularly limited, and is preferably, for example, a size that allows the radical source container 20 to be installed in a room. Further, the shape of the radical source container 20 is not particularly limited, and examples thereof include polyhedrons such as tetrahedron, hexahedron, and octahedron, cylinders, cones, and triangular pyramids.

The radical source container 20 may further store, for example, a radical generating catalyst. The radical generating catalyst is, for example, the same as described above. The radical generating catalyst is released from the radical source container 20 at the same time as the radical source, for example, via the release mechanism 30. On the other hand, the pathogen disposal device 1 of the present invention may further include, for example, a radical generating catalyst container (not shown), and the radical generating catalyst container may store the radical generating catalyst. The material of the radical generating catalyst container is not particularly limited as long as it can store the radical generating catalyst. Specific examples of the radical generating catalyst container include light-shielding chemical tanks made of resin and containers made of resin such as polyethylene terephthalate (PET). The size of the radical generating catalyst container is not particularly limited, and is preferably, for example, a size that allows the radical generating catalyst container 20 to be installed in a room. Further, the shape of the radical generating catalyst container is not particularly limited, and examples thereof include polyhedrons such as tetrahedron, hexahedron, and octahedron, cylinders, cones, and triangular pyramids. The radical generating catalyst container releases the radical generating catalyst to the outside of the pathogen disposal device 1 via the release mechanism 30 to be described below.

The material, size, shape, and the like of the ozone supply mechanism 10, the radical source container 20, and the radical generating catalyst container may be different or the same.

At least one of the radical source or the ozone may be, for example, dissolved in a solvent. The solvent is, for example, the same as described above.

The ozone supply mechanism 10 and the radical source container 20 release the ozone and the radical source to the outside of the pathogen disposal device 1 via the release mechanism 30. Then, the pathogen disposal device 1 of the present invention brings the ozone and the radical source released by the release mechanisms 30 into contact with at least one of an object or an environment containing a pathogen to prepare the antipathogenic agent. The release mechanism 30 may be any device as long as it can release the ozone and the radical source, and the examples thereof include, but not limited to, pipes, nozzles, and fans. Specifically, in the case shown in FIG. 1A, for example, the ozone is released to the outside of the pathogen disposal device 1 via a release mechanism 30a. In the case shown in FIG. 1A, for example, the radical source is released to the outside of the pathogen disposal device 1 via a release mechanism 30b. In this way, when the ozone supply mechanism 10 and the radical source container 20 each include a different release mechanism 30, the release mechanisms 30 may be disposed such that the release port of one release mechanism 30 faces, for example, the release port of the other release mechanism 30. Accordingly, the ozone and the radical source can be efficiently reacted (contacted).

On the other hand, in the case of FIG. 1B, for example, the ozone and the radical source are released to the outside of the pathogen disposal device 1 via the release mechanisms 30c. The pathogen disposal device 1 preferably releases the ozone prior to the radical source in order to increase, for example, the antibacterial and antiviral effects. Further, the ozone supply mechanism 10 and the radical source container 20 may release at least one of the ozone or the radical source a plurality of times. While how the radical source or the ozone is released is not particularly limited, for example, at least one of the radical source or the ozone may be released by spraying into a mist, simultaneously or separately.

The material of the release mechanism 30 is not particularly limited. The size of the release mechanism 30 is not particularly limited, and is preferably, for example, a size that allows the release mechanism 30 to be installed in a room. Further, the shape of the release mechanism 30 is not particularly limited, and examples thereof include polyhedrons such as tetrahedron, hexahedron, and octahedron, cylinders, cones, and triangular pyramids. When the ozone supply mechanism 10 and the radical source container 20 each include a different release mechanism 30, the materials, sizes, shapes, and the like of the respective release mechanisms 30 may be different or the same.

The pathogen disposal device 1 may include, for example, a blower such as a fan for efficiently releasing the ozone and the radical source. The blower may be, for example, provided in at least one of the ozone supply mechanism 10, the radical source container 20, or the release mechanism 30. In addition, the pathogen disposal device 1 may be provided with, for example, a gate for limiting the release of the ozone and the radical source. For example, the gate may be disposed on a surface where the ozone supply mechanism 10 and the radical source container 20 are brought into contact with the release mechanism 30, or may be disposed inside the release mechanism 30.

In the pathogen disposal device 1 of the present invention, the numbers of the ozone supply mechanism 10, the radical source container 20, and the release mechanism 30 are not particularly limited, and may be one or two or more.

Next, a pathogen disposal method using the antipathogenic agent of the present invention will be described. Hereinafter, the pathogen disposal method of the antipathogenic agent of the present invention may be referred to as the "pathogen disposal method of the present invention". The pathogen disposal method of the present invention is carried out, for example, using the pathogen disposal device 1 of FIG. 1, as follows. The pathogen disposal method of the present invention is not limited to the use of the pathogen disposal device 1 in FIG. 1.

The pathogen disposal method of the present invention is a method for disposing a pathogen with an antipathogenic agent including a radical source and ozone, including the steps of: releasing, preparing, and contacting. The antipathogenic agent is not particularly limited, and is, for example, the antipathogenic agent described above. In the pathogen disposal method, the ozone and the radical source are released simultaneously or separately as the releasing step. The ozone and the radical source are, for example, the same as described above. It is preferable that the releasing step involves releasing the ozone, for example, prior to the radical source. In addition, the releasing step involves releasing at least one of the radical source or the ozone, for example, once or a plurality of times. Specifically, for example, in the same manner as described above. Further, in the releasing step, for example, at least one of the radical source or the ozone may be released by spraying into a mist.

Next, in the preparing step, the antipathogenic agent is prepared by the release. Specifically, for example, the ozone and the radical source react to form a reaction product (for example, chlorine dioxide or the like).

Next, as the contacting step, the released ozone and the radical source are brought into contact with at least one of an object or an environment containing a pathogen. The contacting step may be, for example, performed in parallel with the preparing step. That is, in the pathogen disposal step of the present invention, for example, the ozone and the radical source are brought into contact with the object while performing the preparation.

The pathogen disposal method of the present invention may further include, for example, a step of supplying ozone. The ozone supplying step is performed, for example, prior to the releasing step. The ozone supplying step includes, for example, a step of generating ozone. The ozone generating step is, for example, the same as described above. The ozone generation method may generate, for example, ozone gas or ozone water, or both of the ozone gas and the ozone water.

The pathogen disposal method of the present invention may further include, for example, a step of storing a radical source in the radical source container 20. The radical source containing step is performed, for example, prior to the releasing step. The ozone supplying step and the radical source storing step may be performed in parallel or in order. The order is not particularly limited. The way of storing is not particularly limited as long as the radical source can be stored in the radical source container 20.

Further, the radical source storing step may further store the radical generating catalyst in the radical source container. In this case, the pathogen disposal method of the present invention releases the radical generating catalyst to the outside of the radical source container 20 at the same time as the radical source in the releasing step. In the pathogen disposal method of the present invention, the preparing step prepares the antipathogenic agent by catalyzing the generation of radicals from the radical source using the released radical generating catalyst.

Meanwhile, the pathogen disposal method of the present invention may further include, for example, a step of storing a radical generating catalyst. The radical generating catalyst storing step is a step of storing the radical generating catalyst in the radical generating catalyst container. In this case, the pathogen disposal method of the present invention further releases the radical generating catalyst in the releasing step. The radical generating catalyst is released preferably at least one of, for example, at the same time as the radical source, immediately before the release of the radical source, or immediately after the release of the radical source. In the pathogen disposal method of the present invention, the antipathogenic agent is prepared by catalyzing the generation of radicals from the radical source by the released radical generating catalyst in the preparing step.

The pathogen disposal device of the present invention and the pathogen disposal method of the present invention can provide the agent of the present invention that is highly safe and has high antibacterial and virus inactivation effects.

### Examples

Hereinafter, examples of the present invention will be described. However, the present invention is not limited to the following examples.

### Reference Example 1

In the present reference example, it was examined that ozone was decomposed by spraying a radical source after spraying ozone gas in the indoor space.

First, a method for preparing an agent containing a radical source will be described. 5g of sodium chlorite was dissolved in purified water so as to obtain 100 ml of solution, thereby obtaining 40000 ppm sodium chlorite solution (solution A). 0.1g of benzethonium chloride was dissolved in 100 ml of purified water to obtain 100 ml of 1000 ppm solution (solution B). A 0.1 M phosphate NaOH buffer (pH = 9.5) was prepared. 20 ml of diluted 10-fold solution A and 80 ml of buffer were added to 600 ml of purified water of pH 7, and then 80 ml of solution B and purified water were added to obtain 800 ml of solution, thereby obtaining an agent containing a radical source. The agent was a solution containing NaClO₂ and benzethonium chloride. A pH buffer was added to this agent to adjust the pH to 5.35. Hereinafter, the agent containing the radical source is also referred to as a MA-T.

Next, a method for bringing the ozone gas into contact with MA-T will be described. First, an ozone generator (manufacturer model number: AZ291(EASY-30) and brand name: OzoneTechnologies, manufactured by Genetech LLC) was installed in a room of 20 m² and operated for 3 minutes. The ozone concentration in the room 3 minutes after the activation of the ozone generator was 2 to 2.5 ppm when measured using a gas detector tube (KITAGAWA Gas Detector Tube (detector tube name: 182 U ozone, measuring range: 0.025 to 3.0 ppm) manufactured by KOMYO RIKAGAKU KOGYO K.K.). It is to be noted that the indoor space was at normal temperature and pressure. Next, a sprayer was placed in the chamber, and the MAT was sprayed by the sprayer for 5 minutes. After spraying the MA-T, the ozone concentration in the room was measured using a gas detector tube (Kitagawa gas detector tube (detector tube name: 182U ozone, measuring range: 0.025 to 3.0 ppm) manufactured by KOMYO RIKAGAKU KOGYO K.K.).

As a result of the measurement, the ozone concentration in the room after spraying the MA-T was 0.2 ppm.

### Reference Example 2

In the present reference example, it was examined that ozone was decomposed by reacting ozone gas with a radical source in an aqueous solution. As the radical source, the MA-T described in Reference 1 was used.

First, 4 ml of chlorous acid solution (250 ppm) was placed in a cell. Next, 20 ml of ozone generated by an ozone generator in a polystyrene foam box installed in a draft was taken out using a 25 ml-syringe. Then, bubbling by the ozone-containing syringe was performed in the cell containing chlorous acid solution (250 ppm). The absorbance in the cell after bubbling was measured with a spectrophotometer.

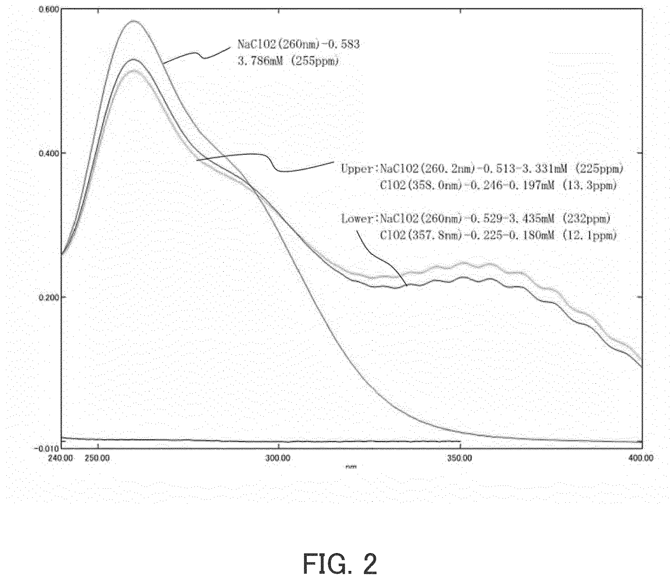

The results of the absorbance are shown in FIG. 2. In the graph of FIG. 2, the vertical axis indicates the absorbance, and the horizontal axis indicates the wavelength. As shown in FIG. 2, generation of chlorine dioxide was observed. That is, it was verified that chlorous acid reduced ozone and chlorous acid became chlorine dioxide in the aqueous solution.

### Example 1

It was examined, by reacting ozone gas with a radical source, that the generated chlorine dioxide exhibited antibacterial and antiviral effects and a deodorizing effect.

### Experimental Example 1

In Experimental Example 1, the bactericidal action by chlorine dioxide was examined using a bacteria A-containing material. The bacteria A are any one of *Staphylococcus aureus*, *Escherichia coli* MV1184, *Streptococcus pyogenes*, *Streptcoccus mutans*, *Porphyromonas gingivalis*, *Treponema denticola*, *Tannerella fosythensis*, Aggregatibacter actinomycetemcomitans, Streptococcus, *Bacillus subtilis*, and *Candida albicans.* The bacteria A-containing material is a liquid medium or an agar medium. The bacterium A may form a biofilm.

Chlorine dioxide generated by bringing ozone gas into contact with a radical source is brought into contact with the bacteria A-containing material. The ozone gas and the radical source were brought into contact with each other in the same manner as in References 1 or 2. After the contact, the bacteria A-containing material was cultured. As a result, it was verified that the chlorine dioxide acts on the bacteria A and exhibits a bactericidal effect.

### Experimental Example 2

In Experimental Example 2, a deodorizing performance test was conducted using smoke derived from tobacco. The deodorizing performance test was conducted in accordance with JEM 1467 "domestic air cleaner" in the Standards of the Japan Electrical Manufacturers' Association. In the measurement, cigarettes were burned while operating a circulator in an acrylic container (1 m in height × 1 m in width × 1 m in depth) with an internal volume of 1 m³ to fill the container with smoke. After all the cigarettes were burned, the circulator was stopped, and the ozone gas and radical source were sprayed in the container by operating a sprayer. The concentrations of three components, namely, ammonia, acetaldehyde, and acetic acid, in the container were measured over 2 hours at regular intervals to trace the change in concentrations. Similarly, formaldehyde vapor was injected into an acrylic container and the formaldehyde concentration in the container was measured over 2 hours at regular intervals to track the change in concentration. The sprayer was operated in "Manual" mode. The malodorous components were measured using detector tubes (Gastec Corporation). The deodorizing performance test verified that the deodorizing effect is achieved by spraying ozone gas and a radical source.

### Experimental Example 3

The ozone gas and radical source were sprayed using a sprayer to measure the deodorizing performance for cigarette odor. First, cigarettes were burned in a room with a 6-tatami mat size to fill the room with smoke at a predetermined concentration. Next, a sprayer was set in the room, and the odor intensity in the room was measured three times, namely, before operating the sprayer, one hour after operating the sprayer, and two hours after operating the sprayer. The sprayer was set near a wall in the room, and the odor was collected at a height of 1 m in the middle of the room. Two circulation fans were set in the room, and they were operated at all times to maintain the air-circulating conditions. The sprayer was operated in "Manual" mode. As a control, a blank test in which the sprayer was not operated was also conducted. The measurement result of the odor intensity verified that the deodorizing effect is achieved by spraying ozone gas and a radical source.

### (Experimental Example 4)

The ozone gas and radical source were sprayed with a sprayer to measure the performance thereof to remove airborne bacteria (general bacteria, fungi). First, a sprayer was set in a room with a 6-tatami mat size, and the concentration of airborne bacteria in the air was measured three times, namely, before operating the sprayer, one hour after operating the sprayer, and two hours after operating the sprayer. The sprayer was set near a wall in the room, and the airborne bacteria were collected at a height of 1 m in the middle of the room. Two circulation fans were set in the room, and they were operated at all times to maintain the air-circulating conditions. The airborne bacteria were measured by a filtration method using a membrane filter. The sprayer was operated in "Manual" mode. The measurement result of the performance to remove airborne bacteria (general bacteria, fungi) verified that the number of airborne bacteria was decreased by spraying ozone gas and a radical source.

### (Experimental Example 5)

Using the chlorine dioxide generated by bringing ozone gas into contact with a radical source, a deodorization test was performed in accordance with "An instrumental analysis implementation manual; A detector tube method, A gas chromatography method" issued by the Certification Standards of Antibacterial Finished Textile Products of the Japan Textile Evaluation Technology Council, with necessary modifications. As a result, the deodorizing effect by the chlorine dioxide was verified.

While the present invention has been described above with reference to illustrative embodiments and examples, the present invention is by no means limited thereto. Various changes and modifications that may become apparent to those skilled in the art may be made in the configuration and specifics of the present invention without departing from the scope of the present invention.

This application claims priority from Japanese Patent Application No. 2020-171087 filed on October 9, 2020. The entire subject matter of the Japanese Patent Application is incorporated herein by reference.

### Industrial Applicability

According to the present invention, it is possible to provide an antipathogenic agent that is highly safe and has high antibacterial and virus inactivation effects. The use of the antipathogenic agent is not particularly limited, and can be used in a wide range of applications.

## Claims

1. An antipathogenic agent, comprising:
a radical source; and
ozone, wherein
the radical source comprises at least one material selected from the group consisting of a halogenous acid, a halite ion, and a halite, and
the radical source and the ozone are brought into contact with at least one material selected from the group consisting of an object containing a pathogen and an environment containing the pathogen, at the same time or at different times.

2. The antipathogenic agent according to claim 1, wherein
the halogenous acid is at least one acid selected from the group consisting of a chlorous acid, a bromous acid, and an iodous acid.

3. The antipathogenic agent according to claim 1 or 2, wherein
the halogenous acid is a chlorous acid.

4. The antipathogenic agent according to any one of claims 1 to 3, further comprising:
a radical generating catalyst, wherein
the radical generating catalyst catalyzes generation of radicals from the radical source.

5. The antipathogenic agent according to any one of claims 1 to 4, wherein
the at least one material selected from the group consisting of the radical source and the ozone is a substance that are configured to be sprayed into a mist at the same time or at the different times.

6. The antipathogenic agent according to any one of claims 1 to 5, wherein
the at least one material selected from the group consisting of the radical source and the ozone is dissolved in a solvent.

7. The antipathogenic agent according to any one of claims 1 to 6, wherein
the ozone is the at least one material selected from the group consisting of ozone gas and ozone water.

8. The antipathogenic agent according to any one of claims 1 to 7, wherein
the pathogen is an infectious pathogen.

9. An antibacterial agent, comprising:
the antipathogenic agent according to any one of claims 1 to 8.

10. An antiviral agent, comprising:
the antipathogenic agent according to any one of claims 1 to 8.

11. A pathogen disposal device configured to dispose a pathogen with an antipathogenic agent comprising a radical source and ozone, the pathogen disposal device comprising:
an ozone supply mechanism;
a radical source container; and
a release mechanism, wherein
the ozone supply mechanism is configured to supply ozone,
the radical source container is configured to store a radical source, the radical source including at least one material selected from the group consisting of a halogenous acid, a halite ion, and a halite,
the ozone supply mechanism and the radical source container are configured to release the ozone and the radical source, respectively, to outside of the device via the release mechanism, and
the ozone and the radical source released by the release mechanism are brought into contact with at least one material selected from the group consisting of an object containing the pathogen and an environment containing the pathogen.

12. The pathogen disposal device according to claim 11, wherein
the halogenous acid comprises at least one material selected from the group consisting of a chlorous acid, a bromous acid, and an iodous acid.

13. The pathogen disposal device according to claim 11 or 12, wherein
the halogenous acid is a chlorous acid.

14. The pathogen disposal device according to any one of claims 11 to 13, wherein
the radical source container is configured to further store a radical generating catalyst, and
the radical generating catalyst is a catalyst that promotes generation of radicals from the radical source.

15. The pathogen disposal device according to any one of claims 11 to 14, further comprising:
a radical generating catalyst container, wherein
the radical generating catalyst container is configured to store the radical generating catalyst,
the radical generating catalyst container is configured to release the radical generating catalyst to the outside of the device via the release mechanism, and
the radical generating catalyst is a catalyst that promotes generation of radicals from the radical source.

16. The pathogen disposal device according to any one of claims 11 to 15, wherein
the ozone supply mechanism is configured to release the ozone prior to the radical source.

17. The pathogen disposal device according to any one of claims 11 to 16, wherein
at least one component selected from the group consisting of the ozone supply mechanism and the radical source container is configured to release at least one material selected from the group consisting of the ozone and the radical source a plurality of times.

18. The pathogen disposal device according to any one of claims 11 to 17, wherein
at least one component selected from the group consisting of the ozone supply mechanism and the radical source container is configured to spray at least one material selected from the group consisting of the ozone and the radical source into a mist to release simultaneously or separately.

19. The pathogen disposal device according to any one of claims 11 to 18, wherein
at least one material selected from the group consisting of the radical source and the ozone is dissolved in a solvent.

20. The pathogen disposal device according to any one of claims 11 to 19, wherein
the ozone is at least one material selected from the group consisting of ozone gas and ozone water.

21. The pathogen disposal device according to any one of claims 11 to 20, wherein
the ozone supply mechanism comprises an ozone generation mechanism.

22. A method for producing an antipathogenic agent comprising a radical source and ozone, comprising:
releasing ozone;
releasing a radical source; and
mixing the ozone and the radical source to prepare the antipathogenic agent, wherein
the radical source comprises at least one material selected from the group consisting of a halogenous acid, a halite ion, and a halite.

23. The method according to claim 22, wherein
the halogenous acid comprises at least one material selected from the group consisting of a chlorous acid, a bromous acid, and an iodous acid.

24. The method according to claim 22 or 23, wherein
the halogenous acid is a chlorous acid.

25. The method according to any one of claims 22 to 24, further comprising:
releasing a radical generating catalyst, wherein
the mixing further mixes the radical source and the radical generating catalyst, and
the radical generating catalyst catalyzes generation of radicals from the radical source.

26. The method according to any one of claims 22 to 25, wherein:
at least one procedure selected from the group consisting of the releasing ozone and the releasing radical source comprises spraying of at least one material selected from the group consisting of the ozone and the radical source, into a mist.

27. The method according to any one of claims 22 to 26, wherein
at least one material selected from the group consisting of the radical source and the ozone is dissolved in a solvent.

28. The method according to any one of claims 22 to 27, wherein
the ozone is at least one material selected from the group consisting of ozone gas and ozone water.

29. The method according to any one of claims 22 to 28, further comprising:
generating ozone, wherein
the generating generates the ozone, and
the releasing releases the generated ozone.

30. An antibacterial treatment method, comprising:
producing an agent; and
contacting the agent with a target to be treated, wherein
the producing an agent is the method for producing an antipathogenic agent according to any one of claims 22 to 29; and
the contacting brings, at least one material selected from the group consisting of a combination of the ozone and the radical source., with the produced antipathogenic agent, into contact with, at least one target selected from the group consisting of an object containing a pathogen and an environment containing the pathogen, so as to perform antibacterial treatment.

31. A virus inactivation method, comprising:
producing an agent; and
contacting the agent with a target to be treated, wherein
the producing an agent is the method for producing an antipathogenic agent according to any one of claims 22 to 29; and
the contacting brings, at least one material selected from the group consisting of a combination of the ozone and the radical source and the produced antipathogenic agent, into contact with, at least one target selected from the group consisting of an object containing a pathogen and an environment containing the pathogen, so as to perform virus inactivation.

32. A pathogen disposal method for disposing a pathogen by an antipathogenic agent comprising a radical source and ozone, comprising:
releasing the ozone and the radical source simultaneously or separately;
preparing the antipathogenic agent by the release; and
contacting at least one material selected from the group consisting of a combination of the ozone and the radical source and the prepared antipathogenic agent with at least one target selected from the group consisting of an object containing the pathogen or an environment containing the pathogen, wherein
the radical source includes at least one material selected from the group consisting of a halogenous acid, a halite ion, and a halite.

33. The pathogen disposal method according to claim 32, wherein
the halogenous acid includes at least one material selected from the group consisting of a chlorous acid, a bromous acid, and an iodous acid.

34. The pathogen disposal method according to claim 32 or 33, wherein
the halogenous acid is a chlorous acid.

35. The pathogen disposal method according to any one of claims 32 to 34,wherein
the releasing the ozone and the radical source releases the ozone prior to the radical source.

36. The pathogen disposal method according to any one of claims 32 to 35, wherein
the releasing the ozone and the radical source releases at least one material selected from the group consisting of the radical source and the ozone, a plurality of times.

37. The pathogen disposal method according to any one of claims 32 to 36, wherein
the releasing the ozone and the radical source sprays at least one material selected from the group consisting of the radical source and the ozone into a mist to release.

38. The pathogen disposal method according to any one of claims 32 to 37, wherein
at least one material selected from the group consisting of the radical source and the ozone is dissolved in a solvent.

39. The pathogen disposal method according to any one of claims 32 to 38, wherein
the ozone is at least one material selected from the group consisting of ozone gas and ozone water.

40. The pathogen disposal method according to any one of claims 32 to 39, further comprising:
supplying the ozone.

41. The pathogen disposal method according to claim 40, wherein
the supplying the ozone comprising:
generating the ozone.

42. The pathogen disposal method according to any one of claims 32 to 41, further comprising:
storing the radical source in a radical source container.

43. The pathogen disposal method according to claim 42, wherein
the storing the radical source further stores a radical generating catalyst in the radical source container,
the releasing the ozone and the radical source releases the radical generating catalyst to outside of the radical source container at the same time as the radical source, and
the preparing the antipathogenic agent catalyzes generation of radicals from the radical source by the released radical generating catalyst as to prepare the antipathogenic agent.

44. The pathogen disposal method according to any one of claims 32 to 43, further comprising:
storing a radical generating catalyst in a radical generating catalyst container, wherein
the releasing the ozone and the radical source further releases the radical generating catalyst, and
the preparing the antipathogenic agent catalyzes generation of radicals from the radical source by the released radical generating catalyst so as to prepare the antipathogenic agent.
